# EUROPEAN PATENT SPECIFICATION

(11) **EP 3 936 161 B1**
(45) Date of publication and mention of the grant of the patent: **07.12.2022**
(21) Application number: 20194287.7
(22) Date of filing: 03.09.2020
(51) Int. Cl.: A61L 9/14, A61L 9/20, B05B 15/70, B60J 5/04, A61L 2/10, A61L 2/18, A61L 2/24, B61D 19/00, A61L 2/00

(54) **VEHICLE DOOR EPIDEMIC PREVENTION DEVICE APPLIED TO PUBLIC TRANSPORTATION VEHICLE**
VORRICHTUNG ZUR VERHINDERUNG VON EPIDEMIEN AN FAHRZEUGTÜREN IN ÖFFENTLICHEN VERKEHRSMITTELN
DISPOSITIF DE PRÉVENTION D'ÉPIDÉMIE POUR PORTE DE VÉHICULE APPLIQUÉ À UN VÉHICULE DE TRANSPORT PUBLIC

(30) Priority: 06.07.2020 TW 109122742
(43) Date of publication of application: 12.01.2022
(73) Proprietor: Sunnder Technology Co. Ltd., 540021 Nantou City, Nantou County (TW)
(72) Inventor: CHANG, CHENG-HSIEN, NANTOU CITY, NANTOU COUNTY 540021 (TW)
(74) Representative: Zeitler Volpert Kandlbinder Patentanwälte Partnerschaft mbB

(56) References cited:
- ES-U- 1 248 117
- KR-A- 20030 032 814
- US-A1- 2014 338 153

## Description

### FIELD OF THE INVENTION

The present invention relates to a vehicle door epidemic prevention device applied to a public transportation vehicle, and more particularly to a vehicle door epidemic prevention device applied to a bus.

### BACKGROUND OF THE INVENTION

In order to reduce air pollution, maintain smooth traffic and save energy, modern cities usually provide many types of public transportation vehicles, such as buses, MRTs, trains, high-speed rails and tourist buses, to transport passengers to designated locations quickly, conveniently, so as to reduce the utilization rate of private vehicles greatly. Every city has a large number of bus systems.

However, because the bus system is used to serve the public, it is easy to accelerate the spread of diseases when an epidemic occurs, such as influenza. At present, the commonly used method of epidemic prevention requires passengers to wear masks when riding on the bus. However, the epidemic prevention effect is really limited. On the other hand, because the door of the bus has an opening and closing design, and the door has many actuating mechanisms, it is impossible to directly apply the design of general disinfection tunnels to the existing buses.

Examples of epidemic prevention devices are disclose by US2014338153 and ES1248117U.

### SUMMARY OF THE INVENTION

The primary object of the present invention is to provide a vehicle door epidemic prevention device applied to a public transportation vehicle, which can avoid the actuating mechanism of the vehicle door and disinfect the passengers passing through the vehicle door to achieve the effect of epidemic prevention.

In order to achieve the object, a vehicle door epidemic prevention device applied to a public transportation vehicle is provided. The vehicle door epidemic prevention device is disposed on the public transportation vehicle. The public transportation vehicle has at least one door. The door has a doorway. The doorway is provided with at least one door panel. The door panel is movable relative to the doorway to be in a closed position or in an open position. When the door panel is in the closed position, the door panel closes the doorway. When the door panel is in the open position, the door panel opens the doorway. The vehicle door epidemic prevention device comprises at least one disinfection unit. The disinfection unit is disposed on the door panel to be moved along with the door panel. The disinfection unit faces the doorway when the door panel is in the open position. The disinfection unit can choose a spray nozzle with the function of spraying disinfectant, or an ultraviolet lamp, or both the spray nozzle and the ultraviolet lamp. The vehicle door epidemic prevention device further comprises at least one control unit. The control unit is connected to the disinfection unit for activating the disinfection unit to perform disinfection toward the doorway when the door panel is in the open position.

When the public transportation vehicle stops at a bus stop and opens the door, the door panel is moved from the closed position to the open position to open the doorway, and the control unit activates the disinfection unit to disinfect the passengers passing through the doorway to achieve the purpose of epidemic prevention. Because the disinfection unit is disposed on the door panel and movable along with the door panel and the disinfection unit faces the doorway when the door panel is in the open position, the disinfection unit will not be interfered by the actuating mechanism of the door, but can disinfect the passengers passing through the doorway completely.

### BRIEF DESCRIPTION OF THE DRAWINGS

FIG. 1 is a planar view of a first embodiment of the present invention;
FIG. 2 is a partial perspective view of the first embodiment of the present invention;
FIG. 3 is a partial exploded view of the first embodiment of the present invention;
FIG. 4 is a block diagram of the first embodiment of the present invention;
FIG. 5 is a schematic view of the first embodiment of the present invention when in use;
FIG. 6 is a partial perspective view of a second embodiment of the present invention;
FIG. 7 is a schematic view showing the operation of the second embodiment of the present invention;
FIG. 8 is a schematic view of the second embodiment of the present invention when in use; and
FIG. 9 is a partial cross-sectional view of a third embodiment of the present invention.

### DETAILED DESCRIPTION OF THE PREFERRED EMBODIMENTS

Embodiments of the present invention will now be described, by way of example only, with reference to the accompanying drawings.

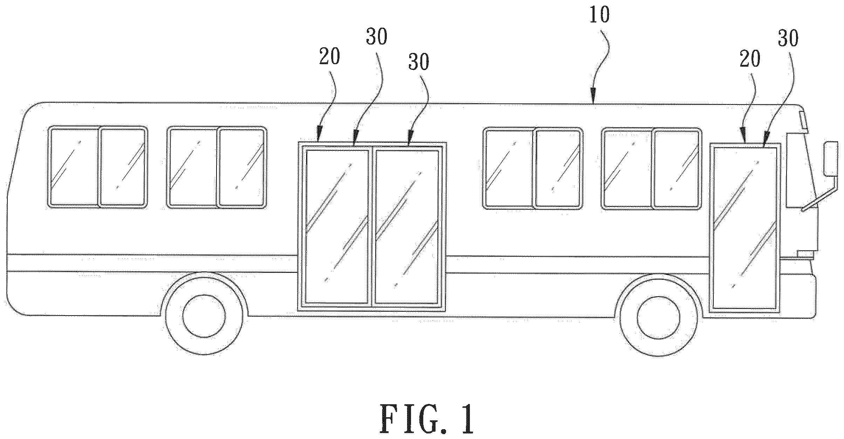

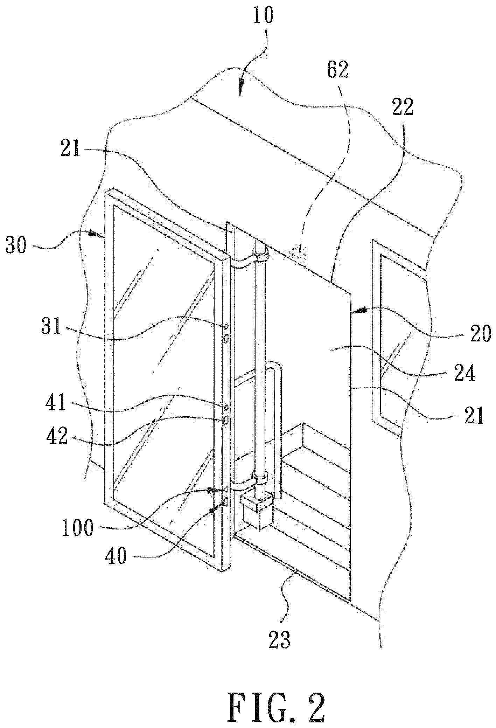

FIG. 1 is a planar view of a first embodiment of the present invention. FIG. 2 is a partial perspective view of the first embodiment of the present invention. The present invention discloses a vehicle door epidemic prevention device 100 applied to a public transportation vehicle. The vehicle door epidemic prevention device 100 may be disposed on the public transportation vehicle 10, especially a bus. The public transportation vehicle 10 has at least one door 20. The door 20 has two side walls 21, and a top wall 22 and a bottom wall 23 connected between the side walls 21. The side walls 21, the top wall 22 and the bottom wall 23 enclose a doorway 24 for passengers or articles to enter and exit. The doorway 24 is provided with at least one door panel 30. The door panel 30 is movable relative to the doorway 24 to be in a closed position or in an open position. In this embodiment, the doorway 24 is provided with a door panel 30 corresponding in size to the doorway 24. The door panel 30 adopts an outward-opening design, and can move laterally relative to the doorway 24. As shown in FIG. 1, when the door panel 30 is in the closed position, the door panel 30 closes the doorway 24. As shown in FIG. 2, when the door panel 30 is in the open position, the door panel 30 is moved laterally away from the doorway 24 to open the doorway 24 so that passengers can freely pass through the doorway 24 to get on and off the vehicle. The outward-opening design is a common knowledge, so it won't be described hereinafter.

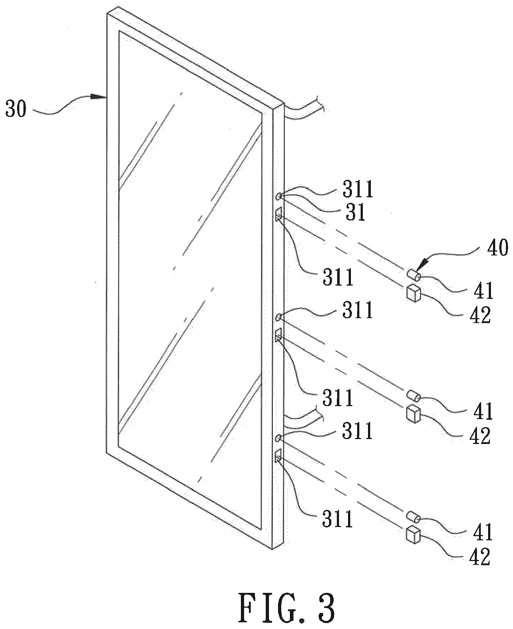

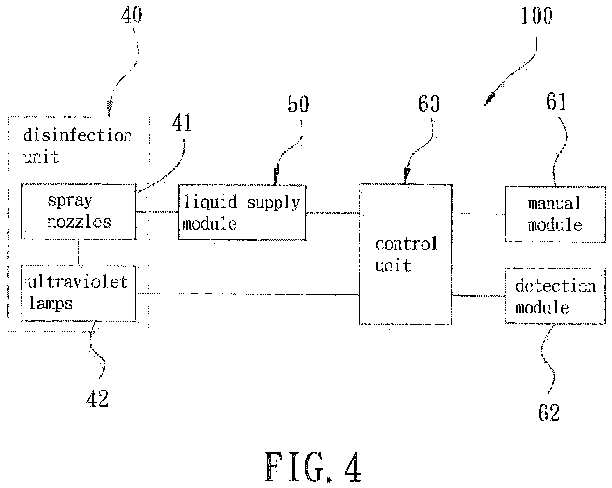

FIG. 3 is a partial exploded view of the first embodiment of the present invention. FIG. 4 is a block diagram of the first embodiment of the present invention. Please refer to FIG. 2, FIG. 3, and FIG. 4. The vehicle door epidemic prevention device 100 includes at least one disinfection unit 40. The disinfection unit 40 is disposed on the door panel 30 to be moved along with the door panel 30. One side of the door panel 30, facing the doorway 24 when the door panel 30 is in the open position, is provided with at least one coupling portion 31. The disinfection unit 40 is arranged on the coupling portion 31, so that the disinfection unit 40 faces the doorway 24 when the door panel 30 is in the open position. The disinfection unit 40 sprays disinfectant or irradiates ultraviolet rays toward the doorway 24 to disinfect the passengers passing through the doorway 24. In order to achieve the above object, the disinfection unit 40 may have at least one spray nozzle 41. The spray nozzle 41 faces the doorway 24 when the door panel 30 is in the open position. The spray nozzle 41 is connected to a liquid supply module 50 through a connecting pipe. The liquid supply module 50 may be a combination of a liquid storage tank and a spray pump motor. The liquid supply module 50 is configured to store a disinfectant, such as hypochlorous acid water or alcohol, so that the disinfectant can be atomized through the spray nozzle 41 and sprayed toward the doorway 24 for disinfection. The disinfection unit 40 may have at least one ultraviolet lamp 42. The preferred wavelength of ultraviolet rays generated by the ultraviolet lamp 42 is between 222 nm and 265 nm. When the door panel 30 is in the open position, the ultraviolet lamp 42 faces the doorway 24, and the ultraviolet lamp 42 irradiates ultraviolet rays toward the doorway 24 for disinfection. Preferably, as shown in this embodiment, the coupling portion 31 of the door panel 30 is composed of a plurality of spaced grooves 311 arranged from top to bottom. The disinfection unit 40 includes a plurality of spray nozzles 41 and a plurality of ultraviolet lamps 42 alternately arranged in the grooves 311 from top to bottom. Thereby, the disinfection unit 40 has the spray nozzles 41 and the ultraviolet lamps 42, so that the disinfection unit 40 can spray the disinfectant and irradiate ultraviolet rays toward the doorway 24 simultaneously. Alternatively, one of the spray nozzles 41 or the ultraviolet lamps 42 can be activated for disinfection according to the needs of use.

Referring to FIG. 4, the vehicle door epidemic prevention device 100 further comprises a control unit 60. The control unit 60 is connected to the disinfection unit 40 for activating the disinfection unit 40 to perform disinfection toward the doorway 24 when the door panel 30 is in the open position. The control unit 60 further includes a manual module 61, such as an activation switch. The manual module 61 may be installed near the driver's seat so that the driver can manually activate the disinfection unit 40. In addition, the control unit 60 further includes a detection module 62, such as a human body sensor, an image recognizer, an ultrasonic sensor, and a thermal image sensor. The detection module 62 is installed near the doorway 24 to automatically activate the disinfection unit 40 when a passenger passing through the doorway 24 is detected. In this embodiment, as shown in FIG. 2, the detection module 62 is installed on the top wall 22 of the door 20.

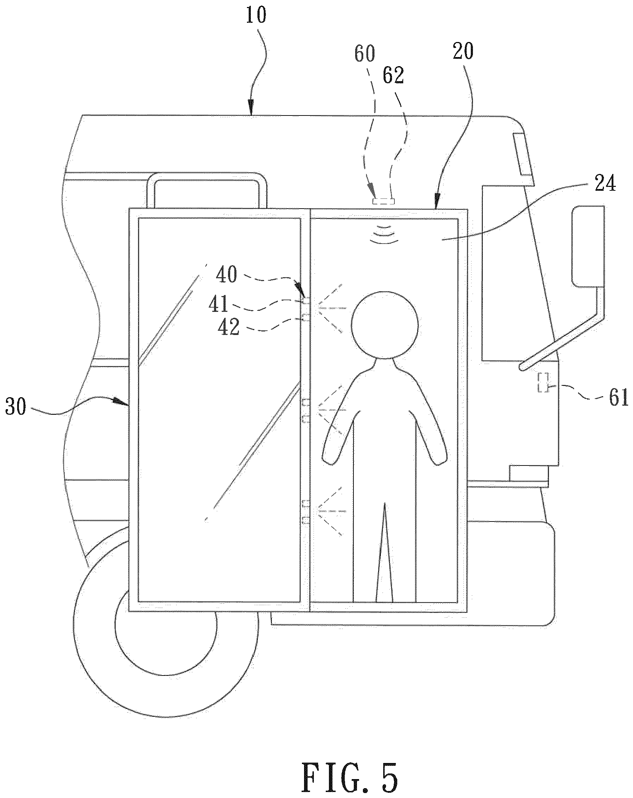

FIG. 5 is a schematic view of the first embodiment of the present invention when in use. When the door panel 30 is in the closed position, the door panel closes the doorway 24 so that passengers or objects cannot get on and off the vehicle, so the disinfection unit 40 will not be activated. When the public transportation vehicle 10 stops at a bus stop and opens the door 20, the door panel 30 is moved from the closed position to the open position to open the doorway 24. At this time, if a passenger or object passes through the doorway 24 to get on and off the vehicle and is detected by the detection module 62, the control unit 60 will automatically activate the disinfection unit 40 and the liquid supply module 50 to atomize the disinfectant through the spray nozzles 41 and spray the disinfectant toward the doorway 24. At the same time, the ultraviolet lamps 42 are activated to irradiate ultraviolet rays toward the doorway 24. Thereby, the passenger or object passing through the doorway 24 can be disinfected to achieve the purpose of epidemic prevention. Because the disinfection unit 40 is disposed on the door panel 30 and faces the doorway 24 when the door panel 30 is in the open position, the disinfection unit 40 will not be interfered by the actuating mechanism of the door 20, but can disinfect the passenger or object passing through the doorway 24 completely. It is worth mentioning that the driver can manually activate the disinfection unit 40 through the manual module 61.

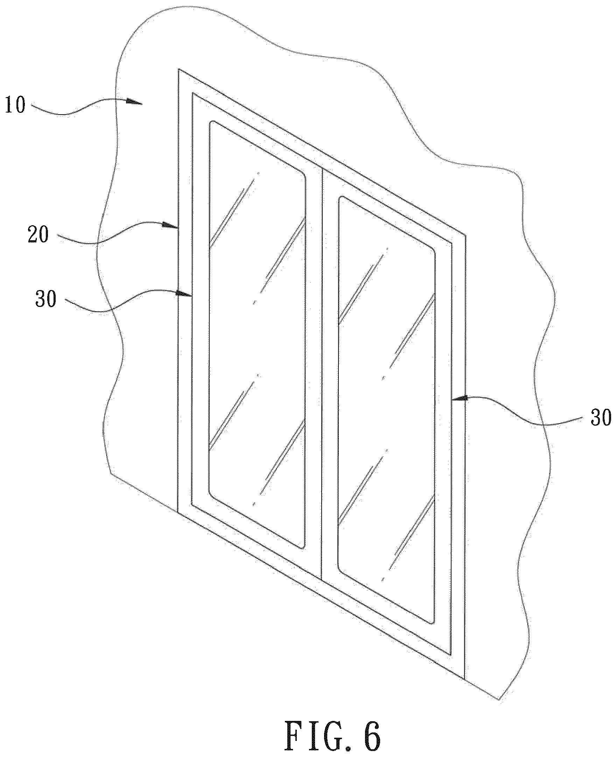

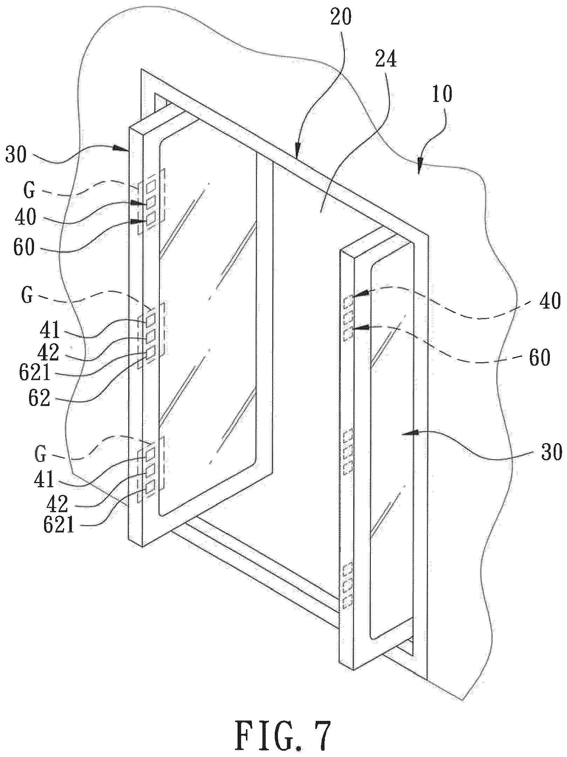

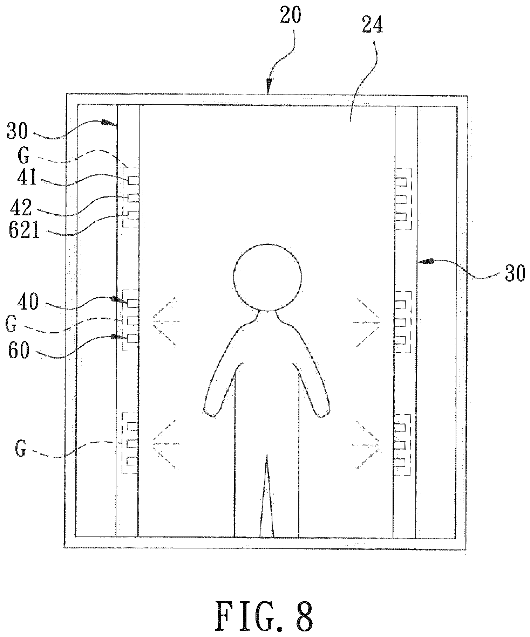

FIG. 6 is a partial perspective view of a second embodiment of the present invention. FIG. 7 is a schematic view showing the operation of the second embodiment of the present invention. FIG. 8 is a schematic view of the second embodiment of the present invention when in use. The second embodiment is substantially similar to the first embodiment with the exceptions described hereinafter. The door 20 is provided with a pair of door panels 30 at the doorway 24. The door panels 30 are designed to open inwardly, and can be rotated relative to the doorway 24. As shown in FIG. 6, when the door panels 30 are in the closed position, the door panels 30 are arranged in parallel to close the doorway 24. As shown in FIG. 6, when the door panels 30 are in the open position, the door panels 30 are rotated 90 degrees to open the doorway 24 so that the passengers can freely pass through the doorway 24 to get on and off the vehicle. The inward-opening design is a common knowledge, so it won't be described hereinafter. Each door panel 30 of the vehicle door epidemic prevention device 100 is provided with a disinfection unit 40, and the disinfection units 40 are located on the relatively inner sides of the door panels 30. Each disinfection unit 40 has a plurality of spaced nozzles 41 and a plurality of spaced ultraviolet lamps 42 arranged from top to bottom on the door panel 30. The detection module 62 has a plurality of spaced sensors 621 arranged from top to bottom on the door panel 30. Preferably, a plurality of groups G are formed from top to bottom, and each group G has a nozzle 41, an ultraviolet lamp 42 and a sensor 621. When the public transportation vehicle 10 stops at a bus stop and opens the door 20, the door panels 30 are moved from the closed position to the open position to open the doorway 24. At this time, if a passenger or object passes through the doorway 24 to get on and off the vehicle, the detection module 62 detects the height of the passenger passing through the doorway 24 by using the sensors 621 and activates the spray nozzle 41 and the ultraviolet lamp 42 corresponding to the group G to spray disinfectant and irradiate ultraviolet rays toward the doorway 24, so as to disinfect the passenger and object passing through the doorway 24. In this way, it is possible to avoid spraying disinfectant or irradiating ultraviolet rays on the face of the passenger, so as to save the consumption of disinfectant and electricity.

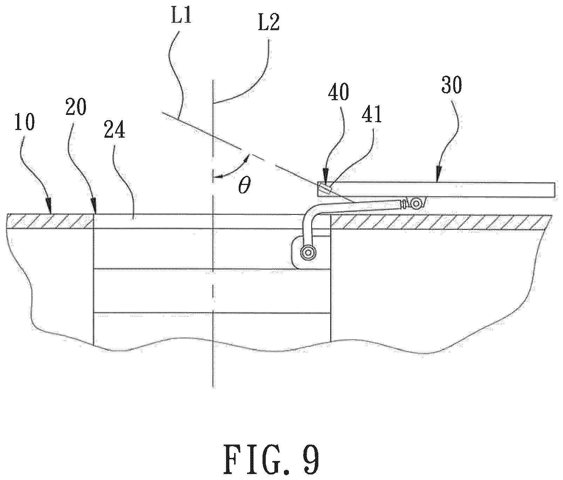

FIG. 9 is a partial cross-sectional view of a third embodiment of the present invention. The third embodiment is substantially similar to the first embodiment with the exceptions described hereinafter. The spraying axis L1 of the spray nozzle 41 is offset toward the outside of the doorway 24, that is, the angle θ between the spraying axis L1 and the central axis L2 of the doorway 24 is less than 90 degrees. Thereby, when the disinfection unit 40 sprays the disinfectant toward the doorway 24, it is possible to prevent the disinfectant from being dispersed into the public transportation vehicle 10.

Although particular embodiments of the present invention have been described in detail for purposes of illustration, various modifications and enhancements may be made without departing from the scope of the appended claims.

## Claims

1. A vehicle door epidemic prevention device (100) applied to a public transportatior vehicle (10), disposed on the public transportation vehicle (10), the public transportation vehicle (10) having at least one door (20), the door (20) having a doorway (24), the doorway (24) being provided with at least one door panel (30), the door panel (30) being movable relative to the doorway (24) to be in a closed position or in an open position, wherein when the door panel (30) is in the closed position, the door panel (30) closes the doorway (24), when the door panel (30) is in the open position, the door panel (30) opens the doorway (24), the vehicle door epidemic prevention device (100)
at least one disinfection unit (40), disposed on the door panel (30) to be moved along with the door panel (30), **characterised in that**, the disinfection unit (40) faces the doorway (24) when the door panel (30) is in the open position;
at least one control unit (60), connected to the disinfection unit (40), for activating the disinfection unit (40) to perform disinfection toward the doorway (24) when the door panel (30) is in the open position.

2. The vehicle door epidemic prevention device (100) as claimed in claim 1, wherein the disinfection unit (40) has at least one spray nozzle (41), the spray nozzle (41) faces the doorway (24) when the door panel (30) is in the open position, the spray nozzle (41) is connected to a liquid supply module (50), the liquid supply module (50) is configured to store a disinfectant, when the door panel (30) is in the open position, the control unit (60) activates the liquid supply module (50) so that the disinfectant is atomized through the spray nozzle (41) and sprayed toward the doorway (24) for disinfection.

3. The vehicle door epidemic prevention device (100) as claimed in claim 1, wherein the disinfection unit (40) has at least one ultraviolet lamp (42), the ultraviolet lamp (42) faces the doorway (24) when the door panel (30) is in the open position, when the door panel (30) is in the open position, the control unit (60) activates the ultraviolet lamp (42) to irradiate ultraviolet rays toward the doorway (24) for disinfection.

4. The vehicle door epidemic prevention device (100) as claimed in claim 1, wherein the disinfection unit (40) has at least one spray nozzle (41), the spray nozzle (41) faces the doorway (24) when the door panel (30) is in the open position, the spray nozzle (41) is connected to a liquid supply module, module (50), the liquid supply module (50) is configured to store a disinfectant; the disinfection unit (40) further has at least one ultraviolet lamp (42), the ultraviolet lamp (42) faces the doorway (24) when the door panel (30) is in the open position; when the door panel (30) is in the open position, the control unit (60) activates the liquid supply module (50) so that the disinfectant is atomized through the spray nozzle (41) and sprayed toward the doorway (24) and the control unit (60) activates the ultraviolet lamp (42) to irradiate ultraviolet rays toward the doorway (24) for disinfection.

5. The vehicle door epidemic prevention device (100) as claimed in claim 2, wherein a spraying axis (L1) of the spray nozzle (41) is offset toward an outside of the doorway (24), and an angle between the spraying axis (L1) and a central (L2) of the doorway (24) is less than 90 degrees.

6. The vehicle door epidemic prevention device (100) as claimed in claim 4, wherein a spraying axis (L1) of the spray nozzle (41) is offset toward an outside of the doorway (24), and an angle between the spraying axis (L1) and a central axis (L2) of the doorway (24) is less than 90 degrees.

7. The vehicle door epidemic prevention device (100) as claimed in claim 1, wherein the control unit (60) includes a detection module (62), the detection module (62) has a plurality of spaced sensors (621) arranged from top to bottom on the door panel (30), when an object passes through the doorway (24), the sensors (621) detect the height of the object.

8. The vehicle door epidemic prevention device (100) as claimed in claim 2, wherein the control unit (60) includes a detection module (62), the detection module (62) has a plurality of spaced sensors (621) arranged from top to bottom on the door panel (30), when an object passes through the doorway (24), the sensors (621) detect the height of the object.

9. The vehicle door epidemic prevention device (100) as claimed in claim 3, wherein the control unit (60) includes a detection module (62), the detection module (62) has a plurality of spaced sensors (621) arranged from top to bottom on the door panel (30), when an object passes through the doorway (24), the sensors (621) detect the height of the object.

10. The vehicle door epidemic prevention device (100) as claimed in claim 4, wherein the control unit (60) includes a detection module (62), the detection module (62) has a plurality of spaced sensors (621) arranged from top to bottom on the door panel (30), when an object passes through the doorway (24), the sensors (621) detect the height of the object.

## Patentansprüche

1. Vorrichtung (100) zum Verhindern von Epidemien an Fahrzeugtüren, die an einem öffentlichen Verkehrsmittel (10) angebracht ist, wobei das öffentliche Verkehrsmittel (10) mindestens eine Tür (20) aufweist, wobei die Tür (20) einen Türdurchgang (24) aufweist, wobei der Türdurchgang (24) mit mindestens einem Türflügel (30) versehen ist, wobei der Türflügel (30) relativ zu dem Türdurchgang (24) beweglich ist, um in einer geschlossenen Position oder in einer offenen Position zu sein, wobei, wenn der Türflügel (30) in der geschlossenen Position ist, der Türflügel (30) den Türdurchgang (24) verschließt, wenn der Türflügel (30) in der offenen Position ist, der Türflügel (30) den Türdurchgang (24) öffnet, wobei die Vorrichtung zum Verhindern von Epidemien an Fahrzeugtüren (100) aufweist:
mindestens eine Desinfektionseinheit (40), die an dem Türflügel (30) angeordnet ist, um zusammen mit dem Türflügel (30) bewegt zu werden, **dadurch gekennzeichnet, dass** die Desinfektionseinheit (40) dem Türdurchgang (24) zugewandt ist, wenn sich der Türflügel (30) in der offenen Position befindet;
mindestens eine Steuereinheit (60), die mit der Desinfektionseinheit (40) verbunden ist, um die Desinfektionseinheit (40) zu betätigen, um eine Desinfektion in Richtung des Türdurchgangs (24) durchzuführen, wenn sich der Türflügel (30) in der offenen Position befindet.

2. Vorrichtung (100) zum Verhindern von Epidemien an Fahrzeugtüren nach Anspruch 1, wobei die Desinfektionseinheit (40) mindestens eine Sprühdüse (41) aufweist, wobei die Sprühdüse (41) dem Türdurchgang (24) zugewandt ist, wenn sich der Türflügel (30) in der offenen Position befindet, wobei die Sprühdüse (41) mit einem Flüssigkeitsversorgungsmodul (50) verbunden ist, wobei das Flüssigkeitsversorgungsmodul (50) zum Speichern eines Desinfektionsmittels ausgebildet ist, wobei die Steuereinheit (60) das Flüssigkeitsversorgungsmodul (50) aktiviert, so dass das Desinfektionsmittel durch die Sprühdüse (41) zerstäubt und zur Desinfektion in Richtung des Türdurchgangs (24) gesprüht wird, wenn sich der Türflügel (30) in der offenen Position befindet.

3. Vorrichtung (100) zum Verhindern von Epidemien an Fahrzeugtüren nach Anspruch 1, wobei die Desinfektionseinheit (40) mindestens eine ultraviolette Lampe (42) aufweist, wobei die ultraviolette Lampe (42) dem Türdurchgang (24) zugewandt ist, wenn sich der Türflügel (30) in der offenen Position befindet, und wobei die Steuereinheit (60) die ultraviolette Lampe (42) aktiviert, um ultraviolette Strahlen zur Desinfektion in Richtung des Türdurchgangs (24) zu strahlen, wenn sich der Türflügel (30) in der offenen Position befindet.

4. Vorrichtung (100) zum Verhindern von Epidemien an Fahrzeugtüren nach Anspruch 1, wobei die Desinfektionseinheit (40) mindestens eine Sprühdüse (41) aufweist, wobei die Sprühdüse (41) dem Türdurchgang (24) zugewandt ist, wenn sich der Türflügel (30) in der offenen Position befindet, wobei die Sprühdüse (41) mit einem Flüssigkeitsversorgungsmodul, Modul (50), verbunden ist, wobei das Flüssigkeitsversorgungsmodul (50) zum Speichern eines Desinfektionsmittels ausgebildet ist; wobei die Desinfektiönseinheit (40) ferner mindestens eine Ultraviolettlampe (42) aufweist, wobei die Ultraviolettlampe (42) dem Türdurchgang (24) zugewandt ist, wenn sich der Türflügel (30) in der offenen Position befindet; wobei die Steuereinheit (60) das Flüssigkeitszufuhrmodul (50) aktiviert, so dass das Desinfektionsmittel durch die Sprühdüse (41) zerstäubt und in Richtung des Türdurchgangs (24) gesprüht wird, und die Steuereinheit (60) die ultraviolette Lampe (42) aktiviert, um ultraviolette Strahlen in Richtung des Türdurchgangs (24) zur Desinfektion zu strahlen, wenn der Türflügel (30) in der offenen Position ist.

5. Vorrichtung (100) zum Verhindern von Epidemien an Fahrzeugtüren nach Anspruch 2, wobei eine Sprühachse (L1) der Sprühdüse (41) in Richtung einer Außenseite des Türdurchgangs (24) versetzt ist und ein Winkel zwischen der Sprühachse (L1) und einer Mittel (L2) des Türdurchgangs (24) weniger als 90 Grad beträgt.

6. Vorrichtung (100) zum Verhindern von Epidemien an Fahrzeugtüren nach Anspruch 4, wobei eine Sprühachse (L1) der Sprühdüse (41) in Richtung einer Außenseite des Türdurchgangs (24) versetzt ist und ein Winkel zwischen der Sprühachse (L1) und einer Mittelachse (L2) des Türdurchgangs (24) weniger als 90 Grad beträgt.

7. Vorrichtung (100) zum Verhindern von Epidemien an Fahrzeugtüren nach Anspruch 1, wobei die Steuereinheit (60) eine Sensoreinheit (62) aufweist, wobei die Sensoreinheit (62) mehrere beabstandete Sensoren (621) aufweist, die von oben nach unten an dem Türflügel (30) angeordnet sind, wobei die Sensoren (621) die Höhe eines Objekts erfassen, wenn das Objekt den Türdurchgang (24) passiert.

8. Vorrichtung (100) zum Verhindern von Epidemien an Fahrzeugtüren nach Anspruch 2, wobei die Steuereinheit (60) eine Sensoreinheit (62) aufweist, wobei die Sensoreinheit (62) mehrere beabstandete Sensoren (621) aufweist, die von oben nach unten an dem Türflügel (30) angeordnet sind, wobei die Sensoren (621) die Höhe eines Objekts erfassen, wenn das Objekt den Türdurchgang (24) passiert.

9. Vorrichtung (100) zum Verhindern von Epidemien an Fahrzeugtüren nach Anspruch 3, wobei die Steuereinheit (60) eine Sensoreinheit (62) aufweist, wobei die Sensoreinheit (62) mehrere beabstandete Sensoren (621) aufweist, die von oben nach unten am Türflügel (30) angeordnet sind, wobei die Sensoren (621) die Höhe eines Objekts erfassen, wenn das Objekt den Türdurchgang (24) passiert.

10. Vorrichtung zum Verhindern von Epidemien an Fahrzeugtüren (100) nach Anspruch 4, wobei die Steuereinheit (60) ein Sensormodul (62) aufweist, wobei das Sensormodul (62) mehrere beabstandete Sensoren (621) aufweist, die von oben nach unten an dem Türflügel (30) angeordnet sind, wobei die Sensoren (621) die Höhe eines Objekts erfassen, wenn das Objekt den Türdurchgang (24) passiert.

## Revendications

1. Dispositif de prévention d'épidémie de porte de véhicule (100) appliqué à un véhicule de transport public (10), disposé sur le véhicule de transport public (10), le véhicule de transport public (10) présentant au moins une porte (20), la porte (20) présentant une baie de porte (24), la baie de porte (24) étant pourvu d'au moins un panneau de porte (30), le panneau de porte (30) étant mobile par rapport à la baie de porte (24) pour être dans une position fermée ou dans une position ouverte, dans lequel, lorsque le panneau de porte (30) est dans la position fermée, le panneau de porte (30) ferme la baie de porte (24), lorsque le panneau de porte (30) est dans la position ouverte, le panneau de porte (30) ouvre la baie de porte (24), le dispositif de prévention d'épidémie de porte de véhicule (100) comprenant :
au moins une unité de désinfection (40), disposée sur le panneau de porte (30) pour être déplacée avec le panneau de porte (30),
**caractérisé en ce que** l'unité de désinfection (40) fait face à la baie de porte (24) lorsque le panneau de porte (30) est dans la position ouverte ;
au moins une unité de commande (60), reliée à l'unité de désinfection (40), pour activer l'unité de désinfection (40) afin d'effectuer une désinfection vers la baie de porte (24) lorsque le panneau de porte (30) est dans la position ouverte.

2. Dispositif de prévention d'épidémie de porte de véhicule (100) selon la revendication 1, dans lequel l'unité de désinfection (40) présente au moins une buse de pulvérisation (41), la buse de pulvérisation (41) fait face à la baie de porte (24) lorsque le panneau de porte (30) est dans la position ouverte, la buse de pulvérisation (41) est reliée à un module d'alimentation en liquide (50), le module d'alimentation en liquide (50) est configuré pour stocker un désinfectant, lorsque le panneau de porte (30) est dans la position ouverte, l'unité de commande (60) active le module d'alimentation en liquide (50) de sorte que le désinfectant est atomisé à travers la buse de pulvérisation (41) et pulvérisé vers la baie de porte (24) pour la désinfection.

3. Dispositif de prévention d'épidémie de porte de véhicule (100) selon la revendication 1, dans lequel l'unité de désinfection (40) présente au moins une lampe à ultraviolets (42), la lampe à ultraviolets (42) fait face à la baie de porte (24) lorsque le panneau de porte (30) est dans la position ouverte, lorsque le panneau de porte (30) est dans la position ouverte, l'unité de commande (60) active la lampe à ultraviolets (42) pour irradier des rayons ultraviolets vers la baie de porte (24) pour la désinfection.

4. Dispositif de prévention d'épidémie de porte de véhicule (100) selon la revendication 1, dans lequel l'unité de désinfection (40) présente au moins une buse de pulvérisation (41), la buse de pulvérisation (41) fait face à la baie de porte (24) lorsque le panneau de porte (30) est dans la position ouverte, la buse de pulvérisation (41) est reliée à un module d'alimentation en liquide (50), le module d'alimentation en liquide (50) est configuré pour stocker un désinfectant ; l'unité de désinfection (40) présente en outre au moins une lampe à ultraviolets (42), la lampe à ultraviolets (42) fait face à la baie de porte (24) lorsque le panneau de porte (30) est dans la position ouverte ; lorsque le panneau de porte (30) est dans la position ouverte, l'unité de commande (60) active le module d'alimentation en liquide (50) de sorte que le désinfectant est atomisé à travers la buse de pulvérisation (41) et pulvérisé vers la baie de porte (24) et l'unité de commande (60) active la lampe à ultraviolets (42) pour irradier des rayons ultraviolets vers la baie de porte (24) pour la désinfection.

5. Dispositif de prévention d'épidémie de porte de véhicule (100) selon la revendication 2, dans lequel un axe de pulvérisation (L1) de la buse de pulvérisation (41) est décalé vers un extérieur de la baie de porte (24), et un angle entre l'axe de pulvérisation (L1) et un axe central (L2) de la baie de porte (24) est inférieur à 90 degrés.

6. Dispositif de prévention d'épidémie de porte de véhicule (100) selon la revendication 4, dans lequel un axe de pulvérisation (L1) de la buse de pulvérisation (41) est décalé vers un extérieur de la baie de porte (24), et un angle entre l'axe de pulvérisation (L1) et un axe central (L2) de la baie de porte (24) est inférieur à 90 degrés.

7. Dispositif de prévention d'épidémie de porte de véhicule (100) selon la revendication 1, dans lequel l'unité de commande (60) inclut un module de détection (62), le module de détection (62) présente une pluralité de capteurs espacés (621) agencés de haut en bas sur le panneau de porte (30), lorsqu'un objet passe à travers la baie de porte (24), les capteurs (621) détectent la hauteur de l'objet.

8. Dispositif de prévention d'épidémie de porte de véhicule (100) selon la revendication 2, dans lequel l'unité de commande (60) inclut un module de détection (62), le module de détection (62) présente une pluralité de capteurs espacés (621) agencés de haut en bas sur le panneau de porte (30), lorsqu'un objet passe à travers la baie de porte (24), les capteurs (621) détectent la hauteur de l'objet.

9. Dispositif de prévention d'épidémie de porte de véhicule (100) selon la revendication 3, dans lequel l'unité de commande (60) inclut un module de détection (62), le module de détection (62) présente une pluralité de capteurs espacés (621) agencés de haut en bas sur le panneau de porte (30), lorsqu'un objet passe à travers la baie de porte (24), les capteurs (621) détectent la hauteur de l'objet.

10. Dispositif de prévention d'épidémie de porte de véhicule (100) selon la revendication 4, dans lequel l'unité de commande (60) inclut un module de détection (62), le module de détection (62) présente une pluralité de capteurs espacés (621) agencés de haut en bas sur le panneau de porte (30), lorsqu'un objet passe à travers la baie de porte (24), les capteurs (621) détectent la hauteur de l'objet.
